Europäisches Patentamt

European Patent Office

Office européen des brevets

(19)

(11) **EP 1 102 987 B1**

(12) **EUROPEAN PATENT SPECIFICATION**

(45) Date of publication and mention
of the grant of the patent:
**17.03.2004 Bulletin 2004/12**

(51) Int Cl.7: **G01N 33/34**

(86) International application number:
**PCT/GB1999/002328**

(21) Application number: **99936767.5**

(22) Date of filing: **04.08.1999**

(87) International publication number:
**WO 2000/008462 (17.02.2000 Gazette 2000/07)**

(54) **METHOD AND APPARATUS FOR MONITORING WATER BALANCE IN A PAPERMACHINE**

VERFAHREN UND VORRICHTUNG ZUR ÜBERWACHUNG DES FEUCHTGLEICHGEWICHTES IN EINER PAPIERMASCHINE

PROCEDE ET APPAREIL DE SURVEILLANCE DE LA QUANTITE RESTANTE D'EAU DANS UNE MACHINE A PAPIER

(84) Designated Contracting States:
**AT DE FI SE**

(30) Priority: **06.08.1998 US 95563 P**

(43) Date of publication of application:
**30.05.2001 Bulletin 2001/22**

(73) Proprietor: **Voith Paper Patent GmbH
89522 Heidenheim (DE)**

(72) Inventor: **LILBURN, David, Andrew
Pittsboro, NC 27312 (US)**

(74) Representative: **Middlemist, Ian Alastair
Wilson Gunn M'Caw,
41-51 Royal Exchange,
Cross Street
Manchester, M2 7BD (GB)**

(56) References cited:
**EP-A- 0 462 703          WO-A-95/34810
DE-A- 4 321 322          US-A- 3 461 030
US-A- 3 607 083**

**Description**

This invention relates to a method and apparatus for monitoring the water balance in a papermachine, in particular in the press section of a papermachine.

In the press section of a papermachine, the wet fibre web is pressed between cylindrical rolls. Press fabrics are passed through the press nips with the web to cushion the web and to absorb water squeezed from the web. A series of press nips acts upon the web before it leaves the press section to pass to the dryer section of the papermachine.

The water removed from the paper web is partly absorbed by the press fabrics (usually one is provided on each face of the paper web), and the remainder is expelled mechanically from the last nip of the press section to be caught in a collection trough. After leaving the press section the press fabric is treated with additional water by showers to clean, condition and lubricate the fabric. The press fabric is then passed over a vacuum area, or one or more slots, such as Uhle boxes, which causes water to migrate to the surface of the fabric where it is removed by mechanical means.

The press section is intended to remove the maximum amount of water without compromising the quality of the paper web produced. The amount of water removed is dependent on the nip pressure, the water absorbtion capacity of the press fabric, and the ability of the press rolls to carry water away from the press nip to the collection trough.

The water absorbtion capacity of the press fabric depends upon the water volume received from the cleaning sprays, the water removal capacity of the vacuum dewatering device, the cleanliness of the press fabric and the design characteristics of the press fabric.

At present it is not possible to know dryness of the paper web as it leaves each press nip or as it finally enters the dryer section. To measure the water content directly requires that the sheet be broken, and the machine be out of use for a costly period of downtime. Indirect measurement of the water content of the web requires that a material balance be determined by measuring water flow from the collection troughs, vacuum dewatering devices, and the water content remaining in the press fabric. The latter is measured using a microwave based moisture meter which is manually pressed into the fabric and moved across the width of the machine. In addition the water content entering press section must be measured or assumed.

Because the carrying out of these material balance procedures require expertise and time, they are not normally carried out except under special circumstances. Consequently, during the normal running of the papermachine there are many unknowns which prevent optimisation of the water removal process.

The unknowns include the effect of press loading on distribution of water flow into the fabric and into the collection trough and overall removal; the effect of the fabric shower flow on the distribution of water flow and overall water removed; the effect of upstream conditions on water removed in the press section; the effect of the paper basis weight on water removal in the press section; the effect of fabric cleanliness on water flow and overall removal; the effect of vacuum levels, dwell time and airflow on water flow and overall removal; the effect of needle jet shower pressures on water flow and overall removal; the effect of roll cover hardness on water flow and overall removal; and the effect of press fabric design on water flow and overall removal.

US Patent 3,655,980 proposes measuring the drainage rate from slurry along the length of a forming wire, using a radiation source and radiation detectors. In US 3,724,957, the concentration of an optically active substance is measured using photoelectric detectors to determine the concentration of pulp and clay in a papermaking slurry, while US 3,860,168 uses a nucleonic detector to monitor the weight of paper sheet. Moisture sensors are used in US 5,093,795, 5,262,955 and 5,286,348. The first two measure the moisture content profile and adjust the moisture content by adding water or steam to the web, and in the other an infra red sensor is used on the web emerging from the last dryer in a papermaking machine.

In US 4,752,356 the slurry is sampled at the wet end of a papermachine to determine the total organic carbon present as a measure of the requirement for cationic additive materials to neutralise anionic contaminants in the papermaking process, and in US 5,330,621 cellulose pulp slurry is continuously analysed to determine elemental constituents, by gamma neutron activation analysis or carbon content analysis. The measurements are not made on the papermachine during a papermaking process.

None of the measurement methods meet the requirements for continuous material balance monitoring during papermachine operation.

It is accordingly an object of this invention to provide a method and apparatus for monitoring the water balance in a papermachine which enables the monitoring to be carried out whilst the machine is operating, and preferably on a continuous or frequent basis.

According to the invention, a method of monitoring the water balance in a papermachine is characterised by using first sensor means to measure the electrical conductivity of the water entrained in one or more press felts on entry to a press means, using second sensor means to measure the electrical conductivity of the water entrained in a paper web on leaving the press means, feeding signals corresponding to the measured values of conductivity to a processor and using the processor to compare the measured electrical conductivities to determine the material balance.

The invention also provides apparatus for monitoring the water balance in a papermachine characterised in that it comprises first sensor means to measure the electrical conductivity of the water entering entrained in one or more press felts on entry to a press means, second sensor means to measure the electrical conductivity of the water entrained in a paper web on leaving the press means, and a processor to which signals corresponding to the measured values of conductivity are sent, to compare the measured electrical conductivities to determine the material balance.

The press means may comprise the entire press section of a papermachine, composed of a series of roller nips, or may comprise a discrete roller nip, where each nip in a press section is for example treated as a separate press means for the purposes of determining the material balance.

The measuring means may each comprise an electrohydrodynamic induction flow meter, as disclosed in US 3,528,287.

If a measurement is made of a specific ion concentration such as $Cl^+$ or $SO_3^{++}$ in each of the input and output streams, a material balance could be determined. Specific ion electrodes have problems with cost, maintenance and excessive specificity. The present invention uses the linear relationship between the concentration of typical papermachine ions and the resulting electrical conductivity and substitutes conductivity for a specific ion concentration. Conductivity measures the total of all ions which will provide a more consistent measurement. Conductivity measurement is simple and requires unsophisticated instrumentation and little day-to-day maintenance.

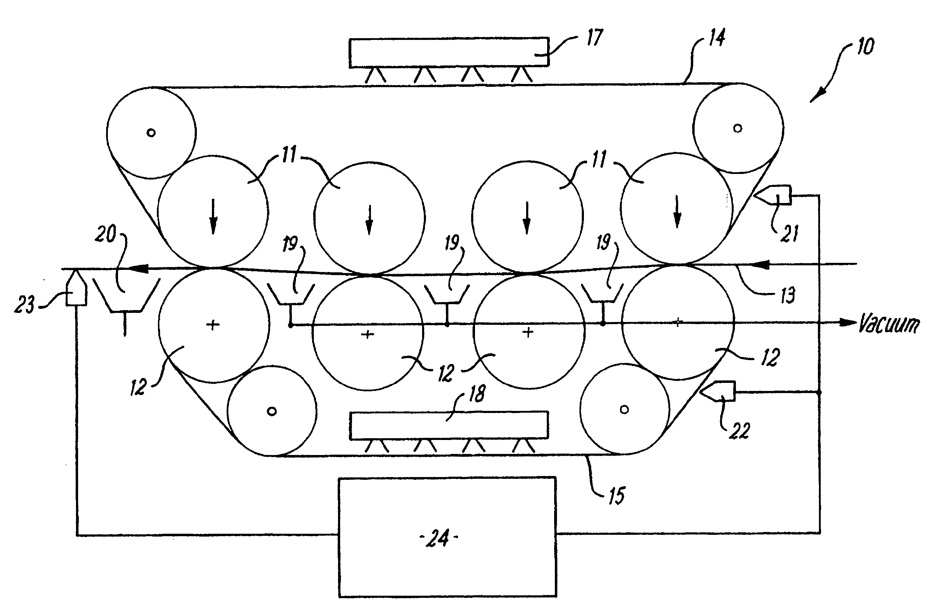

An embodiment of apparatus according to the invention is illustrated by way of example in the accompanying drawing, which is a diagram illustrating the apparatus.

A press section 10 of a papermachine comprises a plurality of nips provided by upper presser rolls 11 and lower anvil rolls 12. A fibrous paper or tissue web 13 enters the section 10 from the right side of the drawing, and leaves towards the left, after passing through the series of nips.

The web 13 is passed through the press section 10 between an upper press felt 14 and a lower press felt 15, which are cleaned by conditioning sprays 17, 18.

Excess moisture is drawn from the felt/web system following each press nip by a vacuum dewatering device 19, such as a respective Uhle box. At the outlet end of the press section 10, excess water is mechanically discharged, and collected by a collecting trough 20.

Means for measuring the electrical conductivity of entrained water are provided in the form of sensors 21, 22 measuring the conductivity of the upper and lower felts respectively at the input end of the press section 10, and sensor 23 measuring the conductivity of the paper or tissue web 13 as it leaves the press section.

Signals corresponding to the measured values of conductivity are fed to a processor 24, which in combination with measuring of other factors as set out in the following Example, calculates a material balance.

EXAMPLE

The material balance may be calculated as follows.

$$\text{Flow gpm} \times \text{Conductivity IN} = \text{Flow} \times \text{Conductivity OUT}$$

In:

Wet Web + Showers

Wet Web gpm calculated = f(Tons/day, % water - (standard or measured))

Wet Web conductivity measured or calculated from previous press nip

Showers gpm - measured or calculated = f(nozzle size, pressure)

Showers conductivity - measured and weight averaged

Out:

Vacuum dewatering flow and conductivity - measured

Press water flow and conductivity - measured

Wet Web gpm = Wet Web in - Vacuum dewatering out - Press water flow

The unknown is now Wet Web conductivity out and is calculated by solving the equation for conductivity.

WWI gpm x WWI cond + Shower gpm x Shower cond =

Uhle gpm x Uhle cond + Press gpm x Press cond + (WWI gpm -

Uhle gpm - Press gpm) x WWO cond

WWI gpm x WWI cond = A

Shower gpm x Shower cond = B

Uhle gpm x Uhle cond = C

Press gpm x Press cond = D

WWI gpm - Uhle gpm - Press gpm = E

A + B = C + D + E x WWO Con

$$\text{WWO cond} = \frac{A + B - C - D}{E}$$

The output (gpm and conductivity) is now known and used as input for the next press nip or as the final exiting conditions.

In addition the conductivity and flow from the collection pans and vacuum removal devices can be analysed to determine what portion is shower water applied to the fabrics and what portion is from water removed from the wet web.

Solids Balance

In:

Wet Web in = WWI gpm x conductivity - from previous stage

Shower in = Shower gpm x conductivity - measured

Out:

Wet Web Out = WWO gpm x conductivity - calculated

Vacuum dewatering = gpm x conductivity - measured

Press Out = gpm x conductivity - measured

Shower water flow = gpm leaving press nip (Xp) + gpm leaving vacuum dewatering (Xu)

Xu = Shower gpm - Xp

Net Web flow out = gpm leaving press nip (Yp) + gpm leaving vacuum dewatering (Yu)

Yu = Net Web flow out - Yp

Uhle box balance

Solids out = Flow x Conductivity

Flow = Xu

Conductivity = Cu

Solids In = Solids from shower water + solids from wet web

Solids from shower water = Shower conc. x Shower water leaving at Uhle box

$= Cs \times Su$

Solids from wet web = Wet web conductivity x Wet web water leaving at Uhle box

$= WWc \times WWu$

$Cu \times Xu = WWc \times WWu + Sc \times Su$

$Xu = WWu + Su$

$WWu = Xu - Su$

$Cu \times Xu = WWc \times (Xu - Su) + Sc \times Su$

$$Su = \frac{Cu \times Xu - WWc \times Xu}{(Sc - WwC)}$$

Su is now the gallons per minute of shower water that is removed in the Uhle box flow. The amount of shower water removed at the press is now the Total Shower flow minus Su. Since the Uhle box flow is known the amount of wet web water removed at the Uhle box is now calculated as the difference between the total and Su. The amount of Wet web water removed at the press can be calculated based on the total press flow if known or a similar material balance at the press nip.

$$\text{Press Cond x Press flow = wet web cond x wet web flow at press +}$$

$$\text{Shower cond x Shower flow at press}$$

$$Cp \times Xp = WWc \times WWp + Sc \times Sp$$

$$\text{Press flow = Wet Web flow + Shower flow}$$

$$Xp = WWp + Sp$$

$$Cp \times (WWp + Sp) = WWc \times WWp + Sc \times Sp$$

$$Wwp = \frac{Sc \times Sp - Cp \times Sp}{(Cp - WWc)}$$

The total flow at the press can now be calculated:

$$Xp = WWp + Sp$$

The material balance (conductivity x flow) enables one to calculate the overall flow in and out of each nip. Comparing the measured versus the calculated flows allow the calculation of a flow error that is then applied to the outgoing sheet to accurately determine sheet consistency. This error is applied to the shower flow but could be applied to the measured weir flows but the resulting sheet consistency would be the same.

The measurement of sufficient flows and conductivities in a press section allows a material balance to be performed that will calculate the exiting % solids leaving each press nip. In addition the distribution between the press nip and press fabric dewatering device of water leaving the wet web can be determined. Finally the distribution of shower water applied to the press fabric between the nip and the dewatering device can be determined.

By measuring these flows and conductivities continuously an on-line water balance can be used to optimise the performance of the press section and its components.

**Claims**

1.    A method of monitoring the water balance in a papermachine is **characterised by** using first sensor means (21,22) to measure the electrical conductivity of the water entrained in one or more press felts (14, 15) on entry to a press means(10), using second sensor means (23)to measure the electrical conductivity of the water entrained in a paper web(13) on leaving the press means (10), feeding signals corresponding to the measured values of conductivity to a processor (24) and using the processor (24) to compare the measured electrical conductivities to determine the material balance.

2.    A method according to claim 1, wherein said first and second sensor means (21, 22, 23) each comprise an electrohydrodynamic induction flow meter.

3.    A method according to claim 1 wherein further signals corresponding to the water flow volumes of felt cleaning showers (17, 18) and the volume of water extracted by suction devices (19, 20) are also fed to the processor for use in the determination of the material balance.

**4.** A method according to claim 1, wherein said press means (10) comprises a complete press section of a papermachine.

**5.** A method according to claim 1, wherein a plurality of press means (10) are provided, comprising each of a plurality of individual roller nips, separate said sensing means (21, 22, 23) being provided for each press means to enable the material balance of each press means to be determined separately.

**6.** Apparatus for monitoring the water balance in a papermachine **characterised in that** it comprises first sensor means (21, 22) to measure the electrical conductivity of water entrained in one or more press felts (14, 15) on entry to a press means (10), second sensor means (23) to measure the electrical conductivity of the water entrained in a paper web (13) on leaving the press means (10), and a processor (24) to which signals corresponding to the measured valves of conductivity are sent, to compare the measured electrical conductivities to determine the material balance.

**7.** Apparatus according to claim 6 wherein said first and second sensor means (21, 22, 23) each comprise an electohydrodynamic induction flow meter.

**8.** Apparatus according to claim 6 further including means for feeding signals corresponding to the water flow volumes of felt cleaning showers (17, 18) and the volume of water extracted by suction devices (19, 20) for use in the determination of the material balance.

**9.** Apparatus according to claim 6 wherein said press means (10) comprises a complete press section of a papermachine.

**10.** Apparatus according to claim 6, wherein a plurality of press means (10) are provided comprising each of a plurality of individual roller nips, and separate sensing means for each press means to enable the material balance of each press means to be determined separately.

**Patentansprüche**

**1.** Ein Verfahren zur Überwachung der Wasserbilanz in einer Papiermaschine ist **gekennzeichnet durch** die Verwendung einer ersten Sensorvorrichtung (21, 22) zur Messung der elektrischen Leitfähigkeit des Wassers, das in einem oder mehreren Preßfilzen (14, 15) beim Eintritt in eine Preßvorrichtung (10) enthalten ist, **durch** die Verwendung einer zweiten Sensorvorrichtung (23) zur Messung der elektrischen Leitfähigkeit des Wassers, das in einer Papierbahn (13) beim Austritt aus der Preßvorrichtung (10) enthalten ist, **durch** das Liefern von Signalen, die den gemessenen Leitfähigkeitswerten entsprechen, an einen Prozessor (24) und **durch** das Verwenden des Prozessors (24) zum Vergleichen der gemessenen elektrischen Leitfähigkeitswerte, um die Materialbilanz zu erstellen.

**2.** Verfahren nach Anspruch 1,
bei dem die erste und die zweite Sensorvorrichtung (21, 22, 23) jeweils einen elektrohydrodynamischen Induktions-Durchflußmesser umfassen.

**3.** Verfahren nach Anspruch 1,
bei dem weitere Signale, die den Wasserdurchflußmengen von Filzreinigungs-Spritzrohren (17, 18) und der durch Saugvorrichtungen (19, 20) extrahierten Wassermenge entsprechen, zur Erstellung der Materialbilanz ebenfalls an den Prozessor gegeben werden.

**4.** Verfahren nach Anspruch 1,
bei dem die Preßvorrichtung (10) eine komplette Pressenpartie einer Papiermaschine umfaßt.

**5.** Verfahren nach Anspruch 1,
bei dem eine Vielzahl von Preßvorrichtungen (10) vorgesehen sind, die jeweils aus einer Vielzahl von einzelnen Walzenspalten bestehen, wobei für jede Preßvorrichtung separate Sensorvorrichtungen (21, 22, 23) vorgesehen sind, damit die Materialbilanz jeder Preßvorrichtung separat erstellt werden kann.

**6.** Vorrichtung zur Überwachung der Wasserbilanz in einer Papiermaschine,

**dadurch gekennzeichnet, daß** sie eine erste Sensorvorrichtung (21, 22) zur Messung der elektrischen Leitfähigkeit des Wassers, das in einem oder mehreren Preßfilzen (14, 15) beim Eintritt in eine Preßvorrichtung (10) enthalten ist, eine zweite Sensorvorrichtung (23) zur Messung der elektrischen Leitfähigkeit des Wassers, das in einer Papierbahn (13) beim Austritt aus der Preßvorrichtung (10) enthalten ist, und einen Prozessor (24) umfaßt, an den Signale, die den gemessenen Leitfähigkeitswerten entsprechen, gegeben werden, um zur Erstellung der Materialbilanz die gemessenen elektrischen Leitfähigkeitswerte zu vergleichen.

**7.** Vorrichtung nach Anspruch 6,
bei der die erste und die zweite Sensorvorrichtung (21, 22, 23) jeweils einen elektrohydrodynamischen Induktions-Durchflußmesser umfassen.

**8.** Vorrichtung nach Anspruch 6,
ferner mit Vorrichtungen zum Liefern von Signalen, die den Wasserdurchflußmengen von Filzreinigungs-Spritzrohren (17, 18) und der durch Saugvorrichtungen (19, 20) extrahierten Wassermenge entsprechen und zur Erstellung der Materialbilanz verwendet werden.

**9.** Vorrichtung nach Anspruch 6,
bei der die Preßvorrichtung (10) eine komplette Pressenpartie einer Papiermaschine umfaßt.

**10.** Vorrichtung nach Anspruch 6,
bei der eine Vielzahl von Preßvorrichtungen (10) vorgesehen ist, die jeweils aus einer Vielzahl von einzelnen Walzenspalten bestehen, und für jede Preßvorrichtung separate Sensorvorrichtungen vorgesehen sind, damit die Materialbilanz jeder Preßvorrichtung separat erstellt werden kann.

**Revendications**

**1.** Procédé de surveillance du bilan hydrique dans une machine à papier, **caractérisé en ce qu'**on utilise des premiers moyens de capteur (21, 22) pour mesurer la conductivité électrique de l'eau entraînée dans un ou plusieurs feutres de presse (14, 15) lors de l'entrée dans un moyen de presse (10), on utilise un deuxième moyen de capteur (23) pour mesurer la conductivité électrique de l'eau entraînée dans une bande de papier (13) lors de la sortie du moyen de presse (10), on délivre des signaux correspondant aux valeurs mesurées de conductivité à un processeur (24), et on utilise le processeur (24) pour comparer les conductivités électriques mesurées afin de déterminer le bilan de matières.

**2.** Procédé selon la revendication 1, dans lequel lesdits premier et deuxième moyens de capteur (21, 22, 23) comprennent chacun un débitmètre à induction électrohydrodynamique.

**3.** Procédé selon la revendication 1, dans lequel d'autres signaux correspondant aux volumes du débit d'eau de rinceurs de feutre (17, 18) et au volume de l'eau extraite par des dispositifs d'aspiration (19, 20) sont également délivrés au processeur pour être utilisés lors de la détermination du bilan de matières.

**4.** Procédé selon la revendication 1, dans lequel ledit moyen de presse (10) comprend une section de presse complète d'une machine à papier.

**5.** Procédé selon la revendication 1, dans lequel une pluralité de moyens de presse (10) sont prévus, comprenant chacune d'une pluralité de lignes de contact individuelles, desdits moyens de capteur (21, 22, 23) séparés étant prévus pour chaque moyen de presse afin de permettre la détermination séparée du bilan de matières de chaque moyen de presse.

**6.** Appareil permettant de surveiller le bilan hydrique dans une machine à papier, **caractérisé en ce qu'**il comprend des premiers moyens de capteur (21, 22) pour mesurer la conductivité électrique de l'eau entraînée dans un ou plusieurs feutres de presse (14, 15) lors de l'entrée dans un moyen de presse (10), un deuxième moyen de capteur (23) pour mesurer la conductivité électrique de l'eau entraînée dans une bande de papier (13) lors de la sortie du moyen de presse (10), et un processeur (24) auquel sont envoyés des signaux correspondant aux valeurs mesurées de conductivité, pour comparer les conductivités électriques mesurées afin de déterminer le bilan de matières.

**7.** Appareil selon la revendication 6, dans lequel lesdits premier et deuxième moyens de capteur (21, 22, 23) comprennent chacun un débitmètre à induction électrohydrodynamique.

8. Appareil selon la revendication 6, incluant en outre un moyen permettant de délivrer des signaux correspondant aux volumes du débit d'eau des rinceurs de feutre (17, 18) et au volume de l'eau extraite par les dispositifs d'aspiration (19, 20) destinés à être utilisés lors de la détermination du bilan de matières.

9. Appareil selon la revendication 6, dans lequel ledit moyen-de presse (10) comprend une section de presse complète d'une machine à papier.

10. Appareil selon la revendication 6, dans lequel une pluralité de moyens de presse (10) sont prévus, comprenant chacune d'une pluralité de lignes de contact individuelles, des moyens de capteur séparés étant prévus pour chaque moyen de presse afin de permettre la détermination séparée du bilan de matières de chaque moyen de presse.

EP 1 102 987 B1